# EUROPEAN PATENT APPLICATION

(11) **EP 4 442 243 A1**
(43) Date of publication of application: **09.10.2024**
(21) Application number: 22901447.7
(22) Date of filing: 02.12.2022
(51) Int. Cl.: A61K 8/34, A61K 8/39, A61Q 5/04

(54) **HAIR TREATMENT METHOD**

(30) Priority: 03.12.2021 JP 2021197231
(71) Applicant: Kao Corporation, Tokyo 103-8210 (JP)
(72) Inventor: EZURE, Mikako, Tokyo 131-8501 (JP); IMAI, Megumi, Tokyo 131-8501 (JP)
(74) Representative: Hoffmann Eitle
(86) International application number: PCT/JP2022/044606
(87) International publication number: WO 2023/101017

(57) **Abstract**

A method for treating hair comprising the following steps (i) and (ii): step (i): applying a hair treatment composition comprising one or more compounds selected from the group consisting of a polyalkylene glycol ether and an alkylene glycol to hair having a wavy curl; and step (ii): carrying out a scrunch operation on the hair in a wet state in which a mass ratio of water to the hair (water/hair) is 0.05 or more and 1 or less, after the step (i).

## Description

### Field of the Invention

The present invention relates to a method for treating hair.

### Background of the Invention

A conventional styling method for hair having a wavy curl has the following problems: the direction of the curl partially curves backward to form a random shape, which not only impairs the beauty, but also causes the curl to come undone from the backward curve portion, reducing the sustainability of the curl.

In addition, conventionally, in setting hair having a wavy curl, a hand technique what is called "scrunch" such as grasping, massaging, or twisting tress has been used (for example, Patent Literatures 1 to 3). However, this hand technique has problems such as the directions of curls being inconsistent, no three-dimensional wavy feel being able to be obtained, and flyaway hair being noticeable during the scrunch.

### Citation List

### Patent Literatures

(Patent Literature 1) JP-A-2015-105271 (paragraphs [0068] and [0070])
(Patent Literature 2) JP-A-2012-131784 (paragraph [0148])
(Patent Literature 3) JP-A-2011-251919 (paragraph [0058])

### Summary of the Invention

The present invention provides a method for treating hair, including the following steps (i) and (ii):
step (i): applying a hair treatment composition containing one or more compounds selected from the group consisting of a polyalkylene glycol ether and an alkylene glycol to hair having a wavy curl; and
step (ii): carrying out a scrunch operation on the hair in a wet state in which a mass ratio of water to the hair (water/hair) is 0.05 or more and 1 or less, after the step (i).

The present invention further provides a composition for improving re-formability of a wavy curl containing one or more compounds selected from the group consisting of a polyalkylene glycol ether and an alkylene glycol.

### Brief Description of Drawing

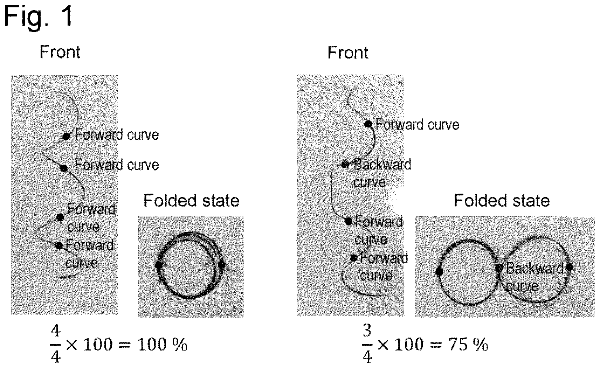

Figure 1 illustrates a method for calculating a spiral rate.

### Detailed Description of the Invention

Because of the above problems, the conventional techniques including Patent Literatures 1 to 3 have the problem of low reproducibility of a wavy curl: these can provide successful styling in some cases and unsuccessful styling in other cases even though the same shampoo, conditioner, and styling agent are used and hair styling is carried out by the same method.

Therefore, the present invention relates to a method for treating hair which can allow hair having a wavy curl to form a spiral shape without a backward curve and to form a curl direction-consistent and three-dimensional wave with high reproducibility during hair styling by scrunch.

The present inventor found that hair styling which meets the above requirements can be acheived by treating hair having a wavy curl with a specific compound, and completed the present invention.

The present invention facilitates to allow hair having a wavy curl to form a spiral shape without a backward curve and form a curl direction-consistent and three-dimensional wave with high reproducibility during hair styling by scrunch.

The method for treating hair according to the present invention includes the following steps (i) and (ii) .

### [Step (i): Application step]

The step (i) is a step of applying a hair treatment composition containing one or more compounds selected from the group consisting of a polyalkylene glycol ether and an alkylene glycol to hair having a wavy curl. The hair treatment composition may be a leave-off formulation such as a shampoo or a conditioner, or a leave-on formulation such as a non-rinse-off type conditioner or hair styling agent.

A target to be treated in the method for treating hair according to the present invention is hair having a wavy curl. The hair having a wavy curl is hair which has several curls between the vicinity of the root and the hair end, and includes both naturally kinky hair and hair that has undergone a hair modification treatment such as a perm.

The hair to be subjected to the step (i) may be dry hair or wet hair, and wet hair is more preferable from the viewpoint of improving the applicability of the hair treatment composition. The wet hair includes not only hair wetted with water, but also hair wetted with water and then towel-dried. When the hair treatment composition is a leave-on type having a high water content such as a hair mist, the hair treatment composition can be preferably applied to any one of dry hair or wet hair.

Examples of the polyalkylene glycol ether contained in the hair treatment composition used in the step (i) include a compound of the following formula (1): 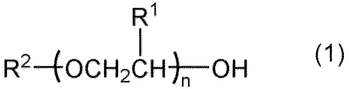
wherein R¹ represents a hydrogen atom or a methyl group,
R² represents an alkyl group having from 1 to 5 carbon atoms, and n represents a number of from 2 to 6.

In the formula (1), R¹ is preferably a hydrogen atom, R² is preferably an alkyl group having from 2 to 4 carbon atoms, and n is preferably a number of from 2 to 3.

Examples of the compound of the formula (1) include diethylene glycol monomethyl ether (methyl carbitol), diethylene glycol monoethyl ether (ethyl carbitol), diethylene glycol monopropyl ether (propyl carbitol), diethylene glycol monobutyl ether (butyl carbitol), triethylene glycol monomethyl ether, and triethylene glycol monoethyl ether, and diethylene glycol monoethyl ether (ethyl carbitol), diethylene glycol monobutyl ether (butyl carbitol), and triethylene glycol monomethyl ether are preferable.

Examples of the alkylene glycol used in the step (i) include ethylene glycol, 1,3-propanediol, 1,3-butanediol, and 3-methyl-1,3-butanediol (isoprene glycol), and 1,3-propanediol, and 3-methyl-1,3-butanediol (isoprene glycol) are preferable.

From the viewpoint of forming a spiral shape without a backward curve, of suppressing flyaway hair and alignment disordering, and of improving the reproducibility of a curl, among the above, one or more selected from the group consisting of diethylene glycol monoethyl ether (ethyl carbitol), diethylene glycol monobutyl ether (butyl carbitol), triethylene glycol monomethyl ether, 1,3-propanediol, and 3-methyl-1,3-butanediol (isoprene glycol) are preferable, one or more selected from the group consisting of diethylene glycol monoethyl ether (ethyl carbitol), diethylene glycol monobutyl ether (butyl carbitol), triethylene glycol monomethyl ether, and 1,3-propanediol are more preferable, one or more selected from the group consisting of diethylene glycol monoethyl ether (ethyl carbitol), diethylene glycol monobutyl ether (butyl carbitol), and triethylene glycol monomethyl ether are further more preferable, one or more selected from the group consisting of diethylene glycol monoethyl ether (ethyl carbitol) and diethylene glycol monobutyl ether (butyl carbitol) are further more preferable, and diethylene glycol monoethyl ether (ethyl carbitol) is even more preferably included.

From the viewpoint of forming a spiral shape without a backward curve and of improving the reproducibility of a curl, the total content of the compounds selected from the group consisting of a polyalkylene glycol ether and an alkylene glycol in the hair treatment composition used in the step (i) is preferably 0.1% by mass or more, more preferably 0.5% by mass or more, further more preferably 1% by mass or more, even more preferably 2% by mass or more, even more preferably 3% by mass or more, even more preferably 4% by mass or more, and even more preferably 5% by mass or more, and from the viewpoint of improving the stability of the formulation (preventing viscosity control from becoming difficult because of large decrease in viscosity during the formulation), the total content is preferably 20% by mass or less, more preferably 18% by mass or less, further more preferably 15% by mass or less, and even more preferably 12% by mass or less.

In particular, when an alkylene glycol is used alone, from the viewpoint of forming a spiral shape without a backward curve and of improving the reproducibility of a curl, the content of the alkylene glycol in the hair treatment composition used in the step (i) is preferably 0.1% by mass or more, more preferably 0.5% by mass or more, and further more preferably 1% by mass or more, and from the viewpoint of improving the stability of the formulation (facilicating viscosity control in view of great decrease in viscosity during preparing the formulation), the content is preferably 10% by mass or less, more preferably 8% by mass or less, and further more preferably 5% by mass or less.

The hair treatment composition preferably contains water as a medium, and can appropriately further contain components conventionally formulated in a hair cosmetic, in accordance with the desired dosage form. Examples thereof include an antidandruff agent, a vitamin, a microbicide, an anti-inflammatory agent, an antiseptic, a chelating agent, a moisturizing agent, a colorant (e.g., a dye or a pigment), a pearlizing agent, a pH adjuster, a fragrance, an ultraviolet absorber, an antioxidant, a photocatalyst, rose water, a surfactant, an organic solvent, a hydrocarbon, a higher alcohol, a thickener, an oil, a silicone, and various film-forming polymers.

From the viewpoint of imparting a smooth feel to the hair or of improving the settability of the hair, the hair treatment composition used in the step (i) can further contain a film-forming polymer. However, the film-forming polymer may weaken the formation of a spiral shape without a backward curve or impair the reproducibility of a curl, and thus the content thereof in the hair treatment composition is preferably 5% by mass or less, more preferably 1% by mass or less, further more preferably 0.5% by mass or less, and even more preferably 0.1% by mass or less, and the hair treatment composition is even more preferably substantially free of a film-forming polymer.

In the present invention, "substantially free" means that the content in the composition is less than 0.01% by mass and preferably 0% by mass.

In addition, in the present invention, the film-forming polymer means a polymer which can form a film on a keratin substance when dried.

Hereinafter, the film-forming polymer will be exemplified.

### <Anionic film-forming polymer>

### • Anionic film-forming polymer including any of crotonic acid, maleic acid, maleic acid monoester, and itaconic acid as constitutional unit:

The anionic film-forming polymer is not particularly limited as long as it includes any of crotonic acid, maleic acid, maleic acid monoester, and itaconic acid as a constitutional unit, and examples thereof include one obtained by copolymerization with one or more of a vinyl ether, a vinyl ester, and a vinyl alcohol. Specific examples thereof include a methyl vinyl ether/alkyl maleate copolymer (e.g., Gantrez ES-225, Gantrez ES-425, and Gantrez SP-215, each manufactured by ISP), a vinyl acetate/crotonic acid copolymer (e.g., Resin 28-1310 manufactured by National Starch), a vinyl acetate/crotonic acid/vinyl neodecanoate copolymer (e.g., Resin 28-2930 manufactured by National Starch), a vinyl acetate/crotonic acid/vinyl propionate copolymer (e.g., Luviset CAP manufactured by BASF), and a vinyl alcohol/itaconic acid copolymer (e.g., KM-118 manufactured by Kuraray Co., Ltd.).

### • Anionic film-forming polymer including (meth)acrylic acid as constitutional unit:

Examples thereof include a vinyl polymer including (meth)acrylic acid as a constitutional unit. Specific examples thereof include an acrylic acid/ethyl acrylate/N-t-butylacrylamide copolymer (e.g., Ultrahold 8 and Ultrahold Strong, each manufactured by BASF), an octylacrylamide/acrylic acid copolymer (e.g., Amphomer V-42 manufactured by National Starch), an acrylate/methacrylate/acrylic acid/methacrylic acid copolymer (e.g., Amerhold DR25 manufactured by Union Carbide Corporation), an acrylates/diacetoneacrylamide copolymer (e.g., Plascize L9540B (product obtained by neutralizing the polymer with 2-amino-2-methyl-1-propyl alcohol) manufactured by GOO Chemical Co., Ltd.), and an acrylates/C1-18 alkyl acrylates/C1-8 alkyl acrylamide copolymer (e.g., Plascize L9909B (product obtained by neutralizing the polymer with 2-amino-2-methyl-1-propyl alcohol) manufactured by GOO Chemical Industry Co., Ltd.).

### • Natural anionic film-forming polymer having either carboxy group or sulfuric acid group:

The natural anionic film-forming polymer is not particularly limited as long as it is a natural polysaccharide having either a carboxyl group or a sulfuric acid group, and examples thereof include carrageenan (e.g., Soageena LX22 and Soageena ML210, each manufactured by Mitsubishi Rayon Co., Ltd.), and xanthan gum (e.g., ECHO GUM T manufactured by Dainippon Pharmaceutical Co., Ltd.).

### • Polyester having either carboxy group or sulfonic acid group:

The polyester is not particularly limited, and examples thereof include a water-dispersible polyester (e.g., AQ38S and AQ55S, each manufactured by the Eastman Kodak Company).

### <Amphoteric film-forming polymer>

The amphoteric film-forming polymer is not particularly limited, and examples thereof include a vinyl polymer. Here, the amphoteric polymer contains both a copolymer including one or more anionic monomers and one or more cationic monomers as constitutional units, and a copolymer including as constitutional units one or more so-called betaine monomers having both an anionic moiety and a cationic moiety in the monomer structure.

Specific examples thereof include a dimethyldiallylammonium/acrylamide/acrylic acid copolymer (e.g., Merquat 3331 manufactured by Calgon), an N-methacryloyloxyethyl-N,N-dimethylammonium-α-N-methylcarboxybetaine/methacrylic acid alkyl ester copolymer (e.g., Yukaformer M-75 and Yukaformer SM, each manufactured by Mitsubishi Chemical Corporation); and an octylacrylamide/acrylate/butylaminoethyl methacrylate copolymer (e.g., Amphomer 28-4910 manufactured by National Starch).

### <Cationic film-forming polymer>

The cationic film-forming polymer is not particularly limited, and examples thereof include a vinyl polymer having one or more selected from the group consisting of a quaternary ammonium structure, a tertiary amine structure, a secondary amine structure, and a primary amine structure, and a natural polysaccharide having one or more selected from the group consisting of a quaternary ammonium structure, a tertiary amine structure, a secondary amine structure and a primary amine structure or a modified product of the natural polysaccharide.

### • Vinyl polymer:

Suitable examples of the vinyl polymer having one or more selected from the group consisting of a quaternary ammonium structure, a tertiary amine structure, a secondary amine structure, and a primary amine structure include a vinyl polymer having, as a constitutional unit, one or more selected from the group consisting of a (meth)acrylic acid ester having a secondary amino group, a tertiary amino group, or a quaternary ammonium group, a (meth)acrylamide having a secondary amino group, a tertiary amino group, or a quaternary ammonium group, an N-substituted allylamine, and an N-substituted allylammonium. Specific examples thereof include an alkylacrylamide/acrylate/alkylaminoalkylacrylamide/polyet hylene glycol methacrylate copolymer (polymer disclosed in Examples of JP-A-1990-180911), an alkylacrylamide/alkylaminoalkylacrylamide/polyethylene glycol methacrylate copolymer (polymer disclosed in Examples of JP-A-1996-291206), a polydimethyldiallylammonium chloride (e.g., Merquat 100 manufactured by Calgon), an acrylamidopropyltrimethylammonium chloride/acrylate copolymer (e.g., Merquat manufactured by Calgon), an acrylamide/dimethyldiallylammonium chloride copolymer (e.g., Merquat 550 and Merquat 2200, each manufactured by Calgon), a methylvinylimidazolium chloride/vinylpyrrolidone copolymer (e.g., Luviquat FC370, Luviquat FC550, Luviquat FC905, and Luviquat HM552, each manufactured by BASF), a vinylpyrrolidone/dimethylaminopropyl methacrylamide copolymer (e.g., Gafquat HS-100 manufactured by ISP), a vinylpyrrolidone/dimethylaminoethyl methacrylate copolymer diethyl sulfate (e.g., Gafquat 734, Gafquat 755N, and Gafquat 755, each manufactured by ISP), a vinyl alcohol/dimethylaminopropyl methacrylamide copolymer (e.g., C-318 manufactured by Kuraray Co., Ltd.), a vinylpyrrolidone/dimethylaminoethyl methacrylate copolymer (e.g., Copolymer 845, Copolymer 937, and Copolymer 958, each manufactured by ISP), a vinylpyrrolidone/alkylamino acrylate/vinylcaprolactam copolymer (e.g., Copolymer VC-713 manufactured by ISP), and a vinyl alcohol/vinylamine copolymer (e.g., VA-120-HCl manufactured by Air Products).

### • Natural polysaccharide or modified product of natural polysaccharide:

Suitable examples of the natural polysaccharide having one or more selected from the group consisting of a quaternary ammonium structure, a tertiary amine structure, a secondary amine structure, and a primary amine structure or a modified product of the natural polysaccharide include a hydroxyethylcellulose/dimethyldiallylammonium chloride copolymer (e.g., Celquat H-100 and Celquat L-200, each manufactured by National Starch), hydroxyethylcellulose/2-hydroxypropyltrimethylammonium chloride (e.g., Polymer JR-400 manufactured by Union Carbide Corporation), guar hydroxypropyltrimonium chloride (e.g., Cosmedia Guar C-261N manufactured by Henkel, or Jaguar C-17 manufactured by Rhone-Poulenc), hydroxypropyl chitosan (e.g., HPCH Liquid HV-10 manufactured by ICHIMARU PHARCOS Co., Ltd.), and a chitosan-dl-pyrrolidone carboxylate (e.g., Kytamer PC manufactured by Union Carbide Corporation).

The hair treatment composition of the present invention can further contain a compound having a reduction action. However, among compounds having a reduction action, a compound having a thiol group or an aldehyde group, a sulfite, and a sulfinic acid may accelerate hair damage and additionally reduce the formation of a spiral shape without a backward curve or the reproducibility of a curl, and thus the hair treatment composition is preferably substantially free of these. In addition, a compound having a reduction action having an ascorbyl group may also cause the above problem depending on the content, and thus the content thereof in the hair treatment composition is preferably less than 1% by mass, more preferably less than 0.5% by mass, further more preferably less than 0.1% by mass, and even more preferably less than 0.05% by mass.

Specific examples of a compound having a reduction action having a thiol group include thioglycolic acid, thiolactic acid, cysteine, cysteamine, homocysteine, glutathione, thioglycerol, thiomalic acid, 2-mercaptopropionic acid, 3-mercaptopropionic acid, thiodiglycol, 2-mercaptoethanol, dithiothreitol, N-acetylcysteine, and an ester and an amide of thioglycolic acid or thiolactic acid.

Specific examples of a compound having a reduction action having an aldehyde group include formaldehyde, 1,3,5-trioxane, paraformaldehyde, and formalin. Similarly, methylene glycol is a compound present in equilibrium in a formaldehyde aqueous solution and is substantially synonymous with formalin and thus shall be included in the definition thereof.

Examples of the sulfite include ammonium sulfite, ammonium hydrogen sulfite, sodium sulfite, sodium hydrogen sulfite, potassium sulfite, and potassium hydrogen sulfite.

Examples of the sulfinic acid include a sulfinate and a benzenesulfinate.

Examples of the compound having a reduction action having an ascorbyl group include ascorbic acid, erythorbic acid, and salts or esters thereof. Specific examples thereof include ascorbic acid, sodium ascorbate, potassium ascorbate, calcium ascorbate, ammonium ascorbate, monoethanolamine ascorbate, diethanolamine ascorbate, erythorbic acid, sodium erythorbate, disodium ascorbyl sulfate, magnesium ascorbyl phosphate, ascorbyl palmitate, ascorbyl stearate, ascorbyl dipalmitate, ascorbyl tetra-2-hexyldecanoate, ascorbyl myristate, ascorbyl laurate, ascorbyl acetate, ascorbyl propionate, ascorbyl tartrate, ascorbyl citrate, ascorbyl succinate, ascorbyl benzoate, potassium (ascorbyl/tocopheryl) phosphate, ethyl ascorbic acid, allantoin ascorbate, chitosan ascorbate, methylsilanol ascorbate, tetradecylhexyl ascorbate, aminopropyl ascorbyl phosphate, ascorbic acid polypeptide, ascorbyl glucoside, and ascorbyl methylsilanol pectinate.

After the step (i), a hair treatment agent containing a compound selected from the group consisting of a polyalkylene glycol ether and an alkylene glycol is applied to hair having a wavy curl, and then any one or more steps of the following step (i-1), step (i-2), and step (i-3) can optionally be carried out.
Step (i-1): rinsing off the hair treatment composition from the hair
Step (i-2): making the hair in a wet state in which a mass ratio of water to the hair (water/hair) is 0.05 or more and 1 or less
Step (i-3): combing the hair

When the hair treatment composition is a leave-off formulation, the step (i-1) of rinsing off the hair treatment composition from the hair is carried out after the step (i). After the step (i-1), from the viewpoint of making the effect of the scrunch operation in the step (ii) more remarkable, the step (i-2) of making the hair in a wet state in which a mass ratio of water to the hair (water/hair) is 0.05 or more and 1 or less can be included. The mass ratio is preferably 0.1 or more, more preferably 0.2 or more, further more preferably 0.3 or more, and even more preferably 0.5 or more, and is preferably 0.9 or less, and more preferably 0.8 or less. Examples of a method for adjusting the degree of wetness in the step (i-2) include water absorption by towel drying and addition of water by using, for example, a spray including water. When the hair after the step (i-1) is in the above wet state, the step (i-2) do not need to be carried out, but the step (i-2) can also be appropriately carried out to adjust the degree of wetness to a more suitable one.

A rough guide to the wet state is shown below.
- The mass ratio (water/hair) is 1: The wet hair after hair washing naturally drains and is in a state where water drips or does not
- The mass ratio (water/hair) is 0.9: The extent that the wet hair after hair washing is lightly wrung by hand
- The mass ratio (water/hair) is 0.7: The extent that a towel is pressed against the wet hair after hair washing to absorb moisture
- The mass ratio (water/hair) is 0.5: The extent that the wet hair after hair washing is rubbed all over with a dry towel to wipe water off
- The mass ratio (water/hair) is 0.3: A state where water is thoroughly wiped off the wet hair after hair washing with a dry towel (to the extent that water cannot be wiped off any more), immediately before the alignment of tresses is disordered. Alternatively, the extent that water is sprayed on dry hair or a styling agent is applied thereto to make the hair feel wet
- The mass ratio (water/hair) is 0.05: The extent that an outbath-treatment is lightly applied to dry hair

When the hair treatment composition is a leave-on formulation, from the viewpoint of making the effect of the scrunch operation more remarkable, the hair after subjected to the step (i) is preferably in a wet state within the above range.

After the step (i), when curls are strongly entangled, the step (i-3) of combing the hair is preferably included before the step (ii), in order to make the flow of the hair consistent and make it easy to carry out the scrunch operation. The combing operation may be carried out by using a tool such as a comb or a brush, or carried out by finger combing.

### [Step (ii): Scrunch step]

The step (ii) is a step of carrying out a scrunch operation on the hair in a wet state in which a mass ratio of water to the hair (water/hair) is 0.05 or more and 1 or less, after the step (i). Here, in the present invention, from the viewpoint of forming a spiral shape without a backward curve, the scrunch operation is preferably one or more operations selected from the group consisting of the following 1) to 3), and any operation of 1) to 3) is more preferably repeated. From the viewpoint of suppressing flyaway hair and alignment disordering, it is preferable to carry out one or more operations selected from the group consisting of 2) and 3), and it is more preferable to carry out the operation of 3). From the viewpoint of forming a wavy curl having a three-dimensional feel and of improving the reproducibility of a curl, it is preferable to carry out one or more operations selected from the group consisting of 1) and 3), and it is more preferable to carry out the operation of 3). During the scrunch operation, a tress may be placed on a towel spread over the palm of a hand.
1) grasping or massaging the hair
2) twisting the hair
3) placing the hair in the palm of a hand, and then lifting up in such a way as to grasp up the hair and releasing the hair

From the viewpoint of making the effect of the scrunch operation more remarkable, it is preferable to carry out the scrunch operation on wet hair in a wet state where the mass ratio of water to the hair (water/hair) is 0.05 or more and 1 or less. The preferable range of this mass ratio is the same as the range described in the step (i-2).

From the viewpoint of forming a wavy curl having a three-dimensional feel without flyaway hair, the number of repetitions of the scrunch operation is preferably 1 time or more, more preferably 3 times or more, and further more preferably 5 times or more, for a handful of tresses, and from the viewpoint of labor reduction, the number is preferably 50 times or less, more preferably 30 times or less, and further more preferably 20 times or less.

By repeating this scrunch operation, hairs are gradually shifted from each other and the individual waves become closer to each other, making it possible to form a wavy curl having a three-dimensional feel and a voluminous feel as a tress.

In the present invention, it is considered that the reason why the wavy curl as described above can be formed by the scrunch operation is that the hair in a wet state treated with a polyalkylene glycol ether and/or alkylene glycol in the step (i) is subjected to the scrunch operation in the step (ii) to make the shape of the tress likely to have a spiral structure. In the present invention, the spiral structure formed by the scrunch operation can be evaluated by the spiral rate represented by the following equation. Spiral rate (%) = number of forward curve points/total number of curl points) × 100 wherein the "number of forward curve points" is the number of points where a certain curl curls in the same direction as half a turn before the curl when a tress is placed from the hair end in such a way as to fold the tress and the direction of the curl is observed from the root side of the tress.

The spiral rate in the present invention expresses the extent of consistency in the spiral shape of the tress as a whole, and does not express the extent of consistency in the shape of each individual hair.

In the present invention, from the viewpoint of forming a wavy curl having a three-dimensional feel without flyaway hair, the spiral rate is preferably 40% or more and 100% or less, more preferably 45% or more and 100% or less, and further more preferably 50% or more and 100% or less.

The hair treatment composition used in the step (i) can improve the effect of the scrunch operation in the step (ii), improve the spiral rate, and improve the re-formability of the wavy curl. Here, "re-formability" means that by finger-combing a wavy curl in which the consistency in the spiral shape of the wavy curl has deteriorated over time because of an external factor such as wind, vibration, or contact to cause flyaway hair or alignment disordering to occur or the three-dimensional appearance to deteriorate, it is possible to improve the consistency in the spiral shape of the wavy curl, reduce flyaway hair or alignment disordering, or improve the three-dimensional appearance. That is, the hair treatment composition is suitable as an agent for improving the effect of a scrunch operation on hair having a wavy curl, a composition for improving the spiral rate, a composition for controlling the spiral structure, and a composition for improving the re-formability of a wavy curl.

Preferable aspects of the present invention with respect to the embodiments described above will be further disclosed below.
<1> A method for treating hair comprising the following steps (i) and (ii):
   step (i): a step of applying a hair treatment composition comprising one or more compounds selected from the group consisting of a polyalkylene glycol ether and an alkylene glycol to hair having a wavy curl; and
   step (ii): a step of carrying out a scrunch operation on the hair in a wet state in which a mass ratio of water to the hair (water/hair) is 0.05 or more and 1 or less, after the step (i).
<2> The method for treating hair according to <1>,
   wherein the polyalkylene glycol ether is preferably a compound of the following formula (1). 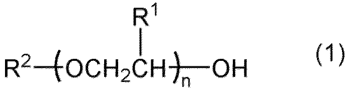
   wherein R¹ represents a hydrogen atom or a methyl group, preferably a hydrogen atom, R² represents an alkyl group having from 1 to 5 carbon atoms, preferably from 2 to 4 carbon atoms, and n represents a number of from 2 to 6, preferably from 2 to 3.
<3> The method for treating hair according to <1> or <2>, wherein the polyalkylene glycol ether is preferably one or more selected from the group consisting of diethylene glycol monomethyl ether (methyl carbitol), diethylene glycol monoethyl ether (ethyl carbitol), diethylene glycol monopropyl ether (propyl carbitol), diethylene glycol monobutyl ether (butyl carbitol), triethylene glycol monomethyl ether, and triethylene glycol monoethyl ether, and more preferably one or more selected from the group consisting of diethylene glycol monoethyl ether (ethyl carbitol), diethylene glycol monobutyl ether (butyl carbitol), and triethylene glycol monomethyl ether.
<4> The method for treating hair according to any one of <1> to <3>, wherein the alkylene glycol is preferably one or more selected from the group consisting of ethylene glycol, 1,3-propanediol, 1,3-butanediol, and 3-methyl-1,3-butanediol (isoprene glycol), and more preferably one or more selected from the group consisting of 1,3-propanediol, and 3-methyl-1,3-butanediol (isoprene glycol).
<5> The method for treating hair according to any one of <1> to <4>, wherein the one or more compounds selected from the group consisting of a polyalkylene glycol ether and an alkylene glycol comprises preferably one or more selected from the group consisting of diethylene glycol monoethyl ether (ethyl carbitol), diethylene glycol monobutyl ether (butyl carbitol), triethylene glycol monomethyl ether, 1,3-propanediol, and 3-methyl-1,3-butanediol (isoprene glycol), more preferably one or more selected from the group consisting of diethylene glycol monoethyl ether (ethyl carbitol), diethylene glycol monobutyl ether (butyl carbitol), triethylene glycol monomethyl ether, and 1,3-propanediol, further more preferably one or more selected from the group consisting of diethylene glycol monoethyl ether (ethyl carbitol), diethylene glycol monobutyl ether (butyl carbitol), and triethylene glycol monomethyl ether, further more preferably one or more selected from the group consisting of diethylene glycol monoethyl ether (ethyl carbitol) and diethylene glycol monobutyl ether (butyl carbitol), and further more preferably diethylene glycol monoethyl ether (ethyl carbitol).
<6> The method for treating hair according to any one of <1> to <5>, wherein a total content of the compounds selected from the group consisting of a polyalkylene glycol ether and an alkylene glycol in the hair treatment composition is preferably 0.1% by mass or more, more preferably 0.5% by mass or more, further more preferably 1% by mass or more, even more preferably 2% by mass or more, even more preferably 3% by mass or more, even more preferably 4% by mass or more, and even more preferably 5% by mass or more, and is preferably 20% by mass or less, more preferably 18% by mass or less, further more preferably 15% by mass or less, and even more preferably 12% by mass or less.
<7> The method for treating hair according to any one of <1> to <6>, wherein the hair treatment composition comprises only an alkylene glycol as the compound selected from the group consisting of a polyalkylene glycol ether and an alkylene glycol, and a content of the alkylene glycol in the hair treatment composition is preferably 0.1% by mass or more, more preferably 0.5% by mass or more, and further more preferably 1% by mass or more, and is preferably 10% by mass or less, more preferably 8% by mass or less, and further more preferably 5% by mass or less.
<8> The method for treating hair according to any one of <1> to <7>, wherein a content of a film-forming polymer in the hair treatment composition is preferably 5% by mass or less, more preferably 1% by mass or less, further more preferably 0.5% by mass or less, and even more preferably 0.1% by mass or less, and the hair treatment composition is even more preferably substantially free of the film-forming polymer.
<9> The method for treating hair according to any one of <1> to <8>, wherein the hair treatment composition is substantially free of a compound having a reduction action selected from the group consisting of a compound having a thiol group or an aldehyde group, a sulfite, and a sulfinic acid.
<10> The method for treating hair according to any one of <1> to <9>, wherein a content of a compound having a reduction action having an ascorbyl group in the hair treatment composition is preferably less than 1% by mass, more preferably less than 0.5% by mass, further more preferably less than 0.1% by mass, and even more preferably less than 0.05% by mass.
<11> The method for treating hair according to any one of <1> to <10>, wherein the hair treatment composition is a leave-off formulation.
<12> The method for treating hair according to <11>, further comprising a step (i-1) of rinsing off the hair treatment composition from the hair after the step (i).
<13> The method for treating hair according to <12>, further comprising, after the step (i-1), a step (i-2) of making the hair in a wet state in which a mass ratio of water to the hair (water/hair) is preferably 0.05 or more, more preferably 0.1 or more, further more preferably 0.2 or more, even more preferably 0.3 or more, even more preferably 0.5 or more, and is preferably 1 or less, more preferably 0.9 or less, and further more preferably 0.8 or less.
<14> The method for treating hair according to any one of <1> to <10>, wherein the hair treatment composition is a leave-on formulation.
<15> The method for treating hair according to any one of <1> to <14>, wherein the step (i) is preferably carried out on preliminarily wetted hair.
<16> The method for treating hair according to any one of <1> to <15>, wherein the hair to be subjected to the step (ii) is in a wet state in which a mass ratio of water to the hair (water/hair) is preferably 0.05 or more, more preferably 0.1 or more, further more preferably 0.2 or more, even more preferably 0.3 or more, and even more preferably 0.5 or more, and is preferably 1 or less, more preferably 0.9 or less, and further more preferably 0.8 or less.
<17> The method for treating hair according to any one of <1> to <16>, wherein the scrunch operation in the step (ii) is preferably one or more operations selected from the group consisting of the following 1) to 3):
   1) grasping or massaging the hair
   2) twisting the hair
   3) placing the hair in a palm of a hand, and then lifting up the hair in such a way as to grasp up the hair and releasing the hair.
<18> The method for treating hair according to <17>, wherein the scrunch operation is preferably one or more operations selected from the group consisting of the 2) and the 3), and more preferably the operation of 3).
<19> The method for treating hair according to <17>, wherein the scrunch operation is preferably one or more operations selected from the group consisting of the 1) and the 3), and more preferably the operation of 3).
<20> The method for treating hair according to any one of <1> to <19>, wherein after the step (i) and before the step (ii) is carried out, the method preferably comprises a step (i-3) of combing the hair.
<21> The method for treating hair according to any one of <1> to <20>, wherein a spiral rate of the hair after treatment represented by the following equation is preferably 40% or more and 100% or less, more preferably 45% or more and 100% or less, and further more preferably 50% or more and 100% or less: spiral rate (%) = number of forward curve points/total number of curl points) × 100
wherein the "number of forward curve points" is the number of points where a certain curl curls in the same direction as half a turn before the curl when a tress is placed from a hair end in such a way as to fold the tress and a direction of the curl is observed from a root side of the tress.
<22> A composition for improving re-formability of a wavy curl, comprising one or more compounds selected from the group consisting of a polyalkylene glycol ether and an alkylene glycol.
<23> An agent for improving an effect of a scrunch operation on hair having a wavy curl, comprising one or more compounds selected from the group consisting of a polyalkylene glycol ether and an alkylene glycol.
<24> A composition for controlling a spiral structure of hair having a wavy curl, comprising one or more compounds selected from the group consisting of a polyalkylene glycol ether and an alkylene glycol.
<25> A composition for improving a spiral rate of hair having a wavy curl, comprising one or more compounds selected from the group consisting of a polyalkylene glycol ether and an alkylene glycol.
<26> An agent for improving an effect of a scrunch operation on hair having a wavy curl, consisting of one or more compounds selected from the group consisting of a polyalkylene glycol ether and an alkylene glycol.
<27> A composition for controlling a spiral structure of hair having a wavy curl, consisting of one or more compounds selected from the group consisting of a polyalkylene glycol ether and an alkylene glycol.
<28>
   A composition for improving a spiral rate of hair having a wavy curl, consisting of one or more compounds selected from the group consisting of a polyalkylene glycol ether and an alkylene glycol.
<29>
   A method for treating hair comprising the following steps (i) and (ii):
   step (i): applying a hair treatment composition comprising one or more compounds selected from the group consisting of a polyalkylene glycol ether and an alkylene glycol to hair having a wavy curl; and
   step (ii): carrying out a scrunch operation on the hair to set a spiral rate of the hair represented by the following equation to 40 or more and 100 or less, after the step (i), spiral rate (%) = number of forward curve points/total number of curl points) × 100
wherein the "number of forward curve points" is the number of points where a certain curl curls in the same direction as half a turn before the curl when a tress is placed from a hair end in such a way as to fold the tress and a direction of the curl is observed from a root side of the tress.
<30>
   A method for treating hair comprising the following steps (i) and (ii):
   step (i): applying a hair treatment composition comprising 3% by mass or more and 18% by mass or less of one or more compounds selected from the group consisting of diethylene glycol monoethyl ether (ethyl carbitol), diethylene glycol monobutyl ether (butyl carbitol), and triethylene glycol monomethyl ether to hair having a wavy curl; and
   step (ii): carrying out one or more scrunch operations selected from the group consisting of the following 1) to 3) on the hair in a wet state in which a mass ratio of water to the hair (water/hair) is 0.3 or more and 1 or less, after the step (i):
      1) grasping or massaging the hair;
      2) twisting the hair; and
      3) placing the hair in a palm of a hand, and then lifting up the hair in such a way as to grasp up the hair and releasing the hair,
wherein after the step (i) and before the step (ii), any one or more selected from the group consisting of step (i-1), step (i-2), and step (i-3) can be optionally carried out:
   step (i-1): rinsing off the hair treatment composition from the hair;
   step (i-2): making the hair in a wet state in which a mass ratio of water to the hair (water/hair) is 0.05 or more and 1 or less; and
   step (i-3): combing the hair.
<31>
   A method for treating hair comprising the following steps (i), (i-1), and (ii):
   step (i): applying a hair treatment composition comprising 5% by mass or more and 15% by mass or less of one or more compounds selected from the group consisting of diethylene glycol monoethyl ether (ethyl carbitol), diethylene glycol monobutyl ether (butyl carbitol), and triethylene glycol monomethyl ether to hair having a wavy curl;
   step (i-1): rinsing off the hair treatment composition from the hair; and
   step (ii): carrying out one or more scrunch operations selected from the group consisting of the following 1) and 3) on the hair in a wet state in which a mass ratio of water to the hair (water/hair) is 0.3 or more and 1 or less, after the step (i):
      1) grasping or massaging the hair; and
      3) placing the hair in a palm of a hand, and then lifting up the hair in such a way as to grasp up the hair and releasing the hair,
wherein after the step (i-1), any one or more selected from the group consisting of step (i-2) and step (i-3) can be optionally carried out:
   step (i-2): making the hair in a wet state in which a mass ratio of water to the hair (water/hair) is 0.05 or more and 1 or less; and
   step (i-3): combing the hair.
<32>
   A composition for improving re-formability of a wavy curl, comprising 3% by mass or more and 18% by mass or less of one or more compounds selected from the group consisting of diethylene glycol monoethyl ether (ethyl carbitol), diethylene glycol monobutyl ether (butyl carbitol), and triethylene glycol monomethyl ether.
<33>
   A composition for improving re-formability of a wavy curl, comprising 5% by mass or more and 15% by mass or less of one or more compounds selected from the group consisting of diethylene glycol monoethyl ether (ethyl carbitol), diethylene glycol monobutyl ether (butyl carbitol), and triethylene glycol monomethyl ether. Examples

### Examples 1 to 10 and Comparative Examples 1 and 2

Samples and comparative samples were prepared by uniformly mixing the components shown in Table 1. Numerical values in the table are active amounts.

**[Table 1]**

| Component (% by mass) | Samples | | | | | | Comparative sample |
|---|---|---|---|---|---|---|---|
| | 1 | 2 | 3 | 4 | 5 | 6 | 1 |
| Diethylene glycol monoethyl ether | 2 | 10 | 20 | - | - | - | - |
| Diethylene glycol monobutyl ether | - | - | - | 10 | - | - | - |
| Triethylene glycol monomethyl ether | - | - | - | - | 10 | - | - |
| 1,3-Propanediol | - | - | - | - | - | 1 | - |
| Water | Balance | Balance | Balance | Balance | Balance | Balance | 100 |

In addition, the following plain shampoo was used for hair treatment.

**[Table 2]**

| Plain shampoo formulation | | |
|---|---|---|
| Raw material name | Component name | Content (% by mass) |
| Purified water | | Balance |
| EMAL 227-pH11 (manufactured by Kao Corporation) | Na laureth (2 mol) sulfate | 15.498 |
| Clewat N (manufactured by Nagase ChemteX Corporation) | Disodium edetate | 0.15 |
| Sodium benzoate solution (35%) | | 0.175 |
| AMINON L-02 (manufactured by Kao Corporation) | Lauramide DEA | 2.28 |
| Phosphoric acid (85%) | | 0.068 |
| NaCl | | 0.8 |
| Fragrance | | 0.4 |

The following evaluations were carried out by using the above samples and comparative samples. Results thereof are shown in Table 3.

### (Tress for evaluation)

Tress A: 0.25 g of Caucasian curly hair (purchased from Kerling International Haarrfabrik, curly hair, 30 cm long, one having 4 or 5 curls from the root to the hair end picked) was used as one tress.

Tress B: 2.0 g of the same hair as the tress A was used as one tress.

### (Hair treatment procedure)

- Step 1: The plain shampoo was applied to the tress of the tress A or the tress B, worked into lather to wash the tress for 1 minute, and then rinsed off with running water for 30 seconds. After rinsing, excess moisture was removed from the hair, and then the samples shown in the Examples were applied to the hair at a bath ratio of 1:1 and let sit for 1 minute.

After that, in Example 1, the tress is held by the root and hung down without rinsing and allowed to drain naturally for 10 seconds. In addition, in Examples 2 to 9 and Comparative Examples 1 and 2, the tress was rinsed in running water for 30 seconds, then held by the root and hung down, and allowed to drain naturally for 10 seconds.

After that, in Example 7, combing (passing a comb from the root to the hair end three times) is carried out.

- Step 2: The hair treated in step 1 is scrunched (*) 10 times. In Comparative Example 2, the hair is not scrunched.
   *Scrunch:
   (a) Imitating the motion of placing the hair in a palm of a hand, lifting up the hair in such a way as to grasp up the hair, and releasing the hair, the motion of pressing the hair against a towel spread on a table from the hair end and lifting up the hair is repeated 10 times at a rate of once per second.
   (b) Imitating the motion of grasping and massaging the hair, the operation of spreading a hand and grasping the hand in such a way as to massage the tress with the tress scooped up from the hair end is repeated 10 times at a rate of once per second.
   (c) Imitating the motion of twisting the hair, the motion of wrapping the tress around a finger and pulling out the finger in the direction of the hair end is repeated 10 times.
- Step 3: The tress is allowed to dry naturally (drying conditions: hanging down the tress and allowing the tress to stand overnight in a laboratory atmosphere (about 25°C, 45 RH))

### (Performance evaluation)

### • Spiral rate:

Five tresses A was subjected to the above hair treatment. After the treatment, as exemplified in Figure 1, the tress is placed from the hair end in such a way as to fold the tress, a curl is counted every half turn of the curl, and when the direction of the curl is observed from the root side of the tress, a point where a certain curl curls in the same direction as half a turn before the curl (forward curve point) is considered to be a beautiful curl created there. A larger number of forward curve points indicates that the curls are more consistent. In accordance with the following equation, the ratio of the total number of forward curve points to the total number of curl points of the tress is defined as the spiral rate. In the evaluation, the average value of the spiral rate of 5 tresses was adopted. Spiral rate (%) = number of forward curve points/total number of curl points) × 100 wherein the "number of forward curve points" is the number of points where a certain curl curls in the same direction as half a turn before the curl when a tress is placed from the hair end in such a way as to fold the tress and the direction of the curl is observed from the root side of the tress.

For example, in the example on the left side of Figure 1, all four curls counted every half turn of the curl are forward curve points, and thus the spiral rate is (4/4) × 100 = 100%, and in the example on the right side of Figure 1, three of the four curls are forward curve points and one is a backward curve point, and thus the spiral rate is (3/4) × 100 = 75%. It is determined that a higher spiral rate indicates that the curl is more uniform.

### • Flyaway hair and alignment disordering score:

The tress B after the hair treatment was photographed, and the number of hairs clearly independent from the tress was determined in a site 3 to 8 cm from the root. A larger number of the hairs indicates that there are more flyaway hairs and the alignment of hairs is disordered.

### • Three-dimensionality:

The tress B after the hair treatment is laid down on a table, a 3.5 g cardboard of 8.5 cm × 21 cm is placed thereon, and the height (cm) from the top surface of the table to the cardboard is measured. A higher height from the top surface of the table to the cardboard indicates that a more firmly curl is formed.

### • Reproducibility 1 (styling with the same finish is always possible):

One tress A is subjected to the above hair treatment, and the direction of a curl is observed. This is run 10 times with the same tress. In each curl generated from the root to the hair end, the number of forward curves in the 10 runs is determined.

A larger number of forward curve points indicates that a larger number of beautiful curls were reproduced. In accordance with the following equation, the ratio of the total number of forward curl points to the total number of all curl points appearing in the 10 runs is evaluated. Number of forward curve points appearing in 10 runs/total number of curl points appearing in 10 runs × 100 (%)

### • Reproducibility 2 (Returning to original even if disturbed):

The tress B after subjected to the hair treatment and drying is combed 10 times to break a wavy curl, and then scrunch treatment is carried out to thereby evaluate the reproducibility of the original wavy curl. Specifically, the state of the tress B after being subjected to the hair treatment and drying is compared with the state of the tress after 10 repetitions of the motion of combing the tress 10 times and then grasping the tress and sliding a hand from the root to the hair end. The tress width at 5 cm, 10 cm, and 15 cm from the root is measured, and the values of [tress width of the tress after 10 repetitions of the motion of combing and then sliding a hand /tress width of the tress before combing] are used to compare the reproducibility of the shape. The closer these values are to 1, the higher the reproducibility is. The table shows the average values of the above values at 5 cm, 10 cm, and 15 cm from the root.

**[Table 3]**

| Treatment | | Examples | | | | | | | | | | Comparative Examples | |
|---|---|---|---|---|---|---|---|---|---|---|---|---|---|
| | | 1 | 2 | 3 | 4 | 5 | 6 | 7 | 8 | 9 | 10 | 1 | 2 |
| Step (i) | Hair treatment agent | Sample 2 | Sample 2 | Sample 1 | Sample 3 | Sample 4 | Sample 5 | Sample 6 | Sample 2 | Sample 2 | Sample 2 | Comparative sample 1 | Sample 2 |
| Step (i-1) | Rinsing | - | Yes | Yes | Yes | Yes | Yes | - | Yes | Yes | Yes | Yes | Yes |
| Step (i-3) | Combing | - | - | - | - | - | - | - | Yes | - | - | - | - |
| Step (ii) | Hair is placed in palm of hand, and then lifted up in such a way as to grasp up hair and released | Yes | Yes | Yes | Yes | Yes | Yes | - | Yes | - | - | Yes | - |
| | Hair is grasped and massaged | - | - | - | - | - | - | - | - | Yes | - | - | - |
| | Hair is twisted | - | - | - | - | - | - | Yes | - | - | Yes | - | - |
| Evaluations | Spiral rate (%) | 48 | 55 | 48 | 46 | 50 | 48 | 45 | 56 | 52 | 42 | 24 | 33 |
| | Flyaway hair and alignment disordering score | 20 | 11 | 15 | 6 | 17 | 22 | 17 | 16 | 20 | 15 | 43 | 31 |
| | Three-dimensionalitv | 1.9 | 2 | 1.9 | 1.9 | 2.1 | 1.8 | 1.9 | 2 | 1.8 | 1.7 | 1.3 | 1.5 |
| | Reproducibility 1 (styling with the same finish is always possible) | 49 | 56 | 48 | 46 | 53 | 45 | 55 | 57 | 49 | 50 | 29 | 36 |
| | Reproducibility 2 (Returning to original even if disturbed) | 1.0/1.1/1 .2 | 0.9/1.0/1 .0 | 1.0/1.0/ 1.8 | 0.9/1.3/ 1.7 | 1.0/1.0/ 1.0 | 1.4/0.9/ 1.0 | 1.2/1.1/0 .9 | 1.2/1.3/ 1.1 | 0.9/0.8/1 .0 | 1.1/1.3/ 1.2 | 1.1/1.8/1.7 | 1.4/1.4/1 .3 |
| | Respective average values at 5/10/15 cm from root | | | | | | | | | | | | |
| | | 1.1 | 1.0 | 1.3 | 1.3 | 1.0 | 1.1 | 1.1 | 1.2 | 0.9 | 1.2 | 1.5 | 1.4 |
| | Overall average value | | | | | | | | | | | | |

## Claims

1. A method for treating hair comprising the following steps (i) and (ii):
step (i): applying a hair treatment composition comprising one or more compounds selected from the group consisting of a polyalkylene glycol ether and an alkylene glycol to hair having a wavy curl; and
step (ii): carrying out a scrunch operation on the hair in a wet state in which a mass ratio of water to the hair (water/hair) is 0.05 or more and 1 or less, after the step (i).

2. The method for treating hair according to claim 1, wherein the scrunch operation in the step (ii) is one or more operations selected from the group consisting of the following 1) to 3):
1) grasping or massaging the hair;
2) twisting the hair; and
3) placing the hair in a palm of a hand, and then lifting up the hair in such a way as to grasp up the hair and releasing the hair.

3. The method for treating hair according to claim 1 or 2, further comprising, after the step (i) and before the step (ii), a step (i-3) of combing the hair.

4. The method for treating hair according to any one of claims 1 to 3, wherein the hair treatment composition is a leave-off formulation.

5. The method for treating hair according to any one of claims 1 to 3, wherein the hair treatment composition is a leave-on formulation.

6. The method for treating hair according to claim 4, further comprising a step (i-1) of rinsing off the hair treatment composition from the hair after the step (i).

7. The method for treating hair according to any one of claims 1 to 6, wherein the step (i) is carried out on preliminarily wetted hair.

8. The method for treating hair according to any one of claims 1 to 7, wherein the hair to be subjected to the step (ii) is in a wet state in which a mass ratio of water to the hair (water/hair) is 0.3 or more and 1 or less.

9. A composition for improving re-formability of a wavy curl, comprising one or more compounds selected from the group consisting of a polyalkylene glycol ether and an alkylene glycol.
